# EUROPÄISCHE PATENTSCHRIFT

(11) **EP 2 934 185 B1**
(45) Veröffentlichungstag und Bekanntmachung des Hinweises auf die Patenterteilung: **12.10.2016**
(21) Anmeldenummer: 13729247.0
(22) Anmeldetag: 15.05.2013
(51) Int. Cl.: A23L 33/105

(54) **CURCUMINSOLUBILISAT**
CURCUMIN SOLUBILISATE
PRODUIT DE SOLUBILISATION DE CURCUMINE

(30) Priorität: 19.12.2012 DE 202012012130 U
(43) Veröffentlichungstag der Anmeldung: 28.10.2015
(73) Patentinhaber: Aquanova AG, 64295 Darmstadt (DE)
(72) Erfinder: BEHNAM, Dariush, 64380 Roßdorf (DE)
(74) Vertreter: Tesch, Sabine
(86) Internationale Anmeldenummer: PCT/EP2013/001427
(87) Internationale Veröffentlichungsnummer: WO 2014/094921

(56) Entgegenhaltungen:
- EP-A1- 2 192 160
- DE-U1-202004 003 241
- "Produkt-Datenblatt Curcumin Wasser-und fettlösliches 6 %iges Curcumin-Solubilisat", , 1. Januar 2009 (2009-01-01), Seiten 1-2, XP055092340, Darmstadt / Germany Gefunden im Internet: URL:http://www.aquanova.de/media/public/pd b/PDB_EW0124_5_NovaSOL Curcumin.pdf [gefunden am 2013-12-09]

## Beschreibung

Curcumin ist ein Gelbwurzelextrakt und seit Jahrhunderten als Gewürz, Lebensmittelfarbstoff (E100), aber auch als Heilmittel in der traditionellen Medizin sowie in den letzten Dekaden in der Schulmedizin als effizienter Wirkstoff in der Immunologie, Osteogenese, Angiogenese, Neurogenese und Karzinogenese durch die Publikation zahlreicher Studienergebnisse global und hinreichend bekannt.

Curcumin wird in zahlreichen Endprodukten (Nahrungsergänzungsmittel), wie zum Beispiel in Kapseln oder in flüssigen, trüben Getränken, in seiner nativen Form (Pulver) oder kombiniert mit Hilfsstoffen wie Öl, Glycerin, Ethanol, Phospholipiden, bzw. Lecithin, Zyclodextrin, Gummi arabicum, Gelantine, Pektinen, Zuckerester von Speisefettsäuren oder Saponinen angeboten. Allerdings ist dabei problematisch, dass diese Formulierungen nicht transparent sind, keine wässrige klare Lösung ergeben und eine extrem geringe Absorption, bzw. Bioverfügbarkeit aufweisen.

Zur Erhöhung der Bioverfügbarkeit ist der Einsatz weiterer Komponenten neben Curcumin zur Schaffung von Trägersystemen wie beispielsweise Emulsionen oder Liposomen bekannt. Während in Emulsionen das Curcumin in einer lipophilen Phase gelöst und in Tropfenform in wäßriger Umgebung stabilisiert wird, kann bei Liposomen das Curcumin in einer Phospholipidschicht gehalten werden. So kann die Bioverfügbarkeit zwar um bis zu 50 % gesteigert werden, jedoch sind derartige Formulierungen wie Liposome mechanisch äußerst instabil und auch nicht resistent gegenüber dem Milieu im Magen.

In der Schulmedizin findet Curcumin insbesondere in Verbindung mit Neurogenese (unter anderem Morbus Alzheimer) und Karzinogenese (Krebs) eine besondere Aufmerksamkeit. Um diesen primär altersbedingten Volkskrankheiten, die inzwischen volkswirtschaftlich bedrohliche Ausmaße angenommen hat, präventiv begegnen zu können, ist die Optimierung der Absorption, bzw. der Bioverfügbarkeit, von Curcumin durch entsprechend geeignete Formulierung, eine Aufgabe der Erfindung. Des Weiteren ist es eine Aufgabe der Erfindung, eine stabil homogene Feinverteilung von Curcumin in den entsprechenden Endprodukten, wie Lebens- und Nahrungsergänzungsmittel zu erreichen.

Die Erfindung stellt eine micellare Curcuminformulierung zur Verfügung, auf deren Basis im Rahmen einer Humanstudie, im Vergleich zu nativem Curcumin, eine mindestens 230fach höhere Bioverfügbarkeit festgestellt werden konnte. Die Erfindung stellt ein Solubilisat bestehend aus Curcumin mit einem Anteil von kleiner oder gleich 10 Gew.-%, bevorzugt kleiner oder gleich 7,5 Gew.-%, besonders bevorzugt 6 Gew.-% und zumindest einem Emulgator mit einem HLB-Wert im Bereich zwischen 13 und 18, nämlich Polysorbat 80 oder Polysorbat 20 oder einer Mischung aus Polysorbat 20 und Polysorbat 80, zur Verfügung, wobei der mittlere Durchmesser der mit Curcumin beladenen Micellen zwischen 5 nm und 40 nm, bevorzugt zwischen 6 nm und 20 nm, besonders bevorzugt zwischen 7 nm und 10 nm liegt.

Damit kann vorteilhafterweise eine hohe Beladung der Micellen mit Curcumin realisiert werden, ohne dass die Micellen aufplatzen und das Curcumin bei Verdünnung mit Wasser als Sediment freigesetzt wird.

Die erfindungsgemäße transparente und vollständig stabil wasserlösliche Curcuminformulierung weist, ohne die oben genannten Hilfsstoffe wie in Weich- und Hartgelatinekapseln, in gelatinefreien Kapseln (hart und/oder weich) und in Getränken oder flüssigen auf Wasser basierenden Endprodukten pH-unabhängig eine stabile Transparenz und darüber hinaus eine deutlich verbesserte Bioverfügbarkeit auf. Produkte mit derartiger Transparenz und Wasserlöslichkeit, aber auch insbesondere so hohet Bioverfügbarkeit der Curcuminformulierung werden seitens der relevanten Industrie dringend für innovative Produkte als Kapselfüllung sowie als transparente Curcumin-Getränke gesucht. Eine Curcuminformulierung, die diesen Anforderungen gerecht wird, existiert bisher nach Kenntnis des Erfinders noch nicht.

Die besonders geringe Größe der Micellen im erfindungsgemäßen Solubilisat führt zu einem klaren und dauerhaft transparenten Produkt. Dazu trägt auch die enge Partikelgrößenverteilung bei, da die der Durchmesserverteilung der Micellen lediglich von etwa 4 nm bis etwa 30 nm reicht

Die Partikelgrößenverteilung der Micellen wurde gemessen nach dem Prinzip der dynamischen Lichtstreuung in 180° Rückstreuanordung mit Laserlicht der Wellenlänge 780 nm. Durch die kleinen Partikelgrößen wird vorteilhafterweise die Ausbildung einer insbesondere für die Wahrnehmung mit dem menschlichen Auge klaren Flüssigkeit erreicht. Die Klarheit des Solubilisats läßt sich auch durch seine geringe Trübung darstellen.

Dazu wird folgende Arbeitshypothese angewandt: Je klarer eine wässrige Verdünnung eines Solubilisats oder einer anderen Formulierung von Curcumin ist, desto besser ist dessen Solubilisation. Je besser die Solubilisation, desto besser ist die Bioverfügbarkeit.

Das erfindungsgemäße Solubilisat zeichnet sich des Weiteren dadurch aus, dass die Gesamt-Curcuminoid-Konzentration im humanen Blutplasma gemessen eine Stunde nach oraler Gabe von 500 mg Curcumin in Form des Solubilisats bei etwa 500 ng Curcuminoid pro mL Plasma ± 100 ng Curcuminoid pro mL Plasma liegt. Demgegenüber werden bei oraler Aufnahme von nativem Curcumin in Pulverform nach einer Stunde lediglich von etwa 1,3 ng Curcuminoid pro mL Plasma erreicht.

Die diesen Werten zugrunde liegende Humanstudie wurde an 24 gesunden Personen im Alter zwischen 19 und 29 Jahren durchgeführt, die einmalig oral eine Dosis von 500 mg nativem Curcumin oder Curcumin in Form des erfindungsgemäßen Solubilisats erhielten. Zu verschiedenen Zeitpunkten innerhalb von 24 Stunden nach der Curcumingabe wurden Blutproben entnommen. Um den möglichen Einfluß parallel verdauter Nahrung zu minimieren, wurden den Testpersonen standardisierte Mahlzeiten serviert. Eine Stunde nach Einnahme des Curcumins in nativer Form wurden weniger als 1 nmol/L Plasma gemessen, 8 Stunden nach der Einnahme 2,4 nmol/L und 24 Stunden nach der Einnahme 2,4 nmol/L. Demgegenüber wurden bei Curcumin aus dem erfindungsgemäßen Solubilisat eine Stunde nach der Einnahme 1,964 nmol/L Plutplasma gemessen, 8 Stunden nach der Einnahme bereits 307,1 nmol/L und 24 Stunden nach der Einnahme 67,7 nmol/L. Dabei wurde ein Solubilisat mit 66,5 Gew.-% Curcumin verwendet (als Handelsware bezeichnet als "6% NovaSOL curcumin" der Anmelderin). Es zeigen sich also Steigerungen der Curcumin-Konzentration im Blutplasma um einen Faktor zwischen 36 und 2800 durch die Formulierung als erfindungsgemäßes Solubilisat gegenüber nativem Curcumin.

Über einen Zeitraum von 24 Stunden gemessen betrug die Fläche unter der Kurve der gesamten Curcumin-Konzentration im Blutplasma (area under the total curcumin plasma concentration-time curve AUC) 42,6 nmol h / L bei Gabe von nativem Curcumin und 9821,4 nmol h /L bei Gabe des erfindungsgemäßen Solubilisats. Etwas verallgemeinert läßt sich sagen, daß die plasma AUC über 24 Stunden des erfindungsgemäßen Curcumin-Solubilisats im Bereich von etwa 9.500 bis etwa 10.000 nmol h /L liegt.

Demnach ist die Bioverfügbarkeit des 66,5 %igen Curcuminssolubilisats deutlich besser als die der nativen Form. Betrachtet als plasma AUC über 24 Stunden ist die Bioverfügbarkeit durch die erfindungsgemäße Formulierung des Solubilisats etwa 230fach erhöht.

Diese läßt sich bereits an der besonders geringen Trübung des Solubilisats ablesen, welche sich als eine Art Kenngröße für die Bioverfügbarkeit verstehen läßt. Die Trübung des erfindungsgemäßen Solubilisats ist kleiner als 30 FNU, bevorzugt kleiner als 20 FNU, und liegt besonders bevorzugt im Bereich zwischen 0,5 FNU und 2 FNU, gemessen durch Streulichtmessung mit Infrarotlicht nach den Vorschriften der Norm ISO 7027 bei einer Verdünnung des Solubilisats im Verhältnis 1:1000 in Wasser.

Diese geringe Trübung behält das erfindungsgemäße Solubilisat auch nach 24 Stunden Lagerung bei 21°C und pH 7 ebenso wie nach 1 Stunde Lagerung bei 37°C und pH 1,1, also einerseits unter Lagerbedingungen bei Raumtemperatur in wäßriger Verdünnung und andererseits bei der Passage des Magens. Daher liegt das Curcumin im erfindungsgemäßen Solubilisat nach jetzigem Verständnis des Erfinders auch nach Passage des Magens noch in Form der stabilen, sehr kleinen Micellen vor und kann daher besonders gut im weiteren Verdauungstrakt aufgenommen werden.

Zur experimentellen Bestimmung der Trübung werden die Trübungsmessgeräte mit einer Standardsuspension kalibriert. Die Anzeige erfolgt somit nicht in Form der gemessenen Lichtintensität, sondern als Konzentration der Kalibriersuspension. Bei der Messung einer beliebigen Suspension bedeutet also die Anzeige, dass die betreffende Flüssigkeit die gleiche Lichtstreuung verursacht wie die Standardsuspension der angezeigten Konzentration. Der international festgelegte Trübungsstandard ist Formazin. Zu den geläufigsten Einheiten gehört die Angabe "FNU", das heißt "Formazine Nephelometric Units". Dies ist die beispielsweise in der Wasseraufbereitung verwendete Einheit für die Messung bei 90° gemäss den Vorschriften der Norm ISO 7072. Die Trübung des erfindungsgemäßen Solubilisats ist kleiner als 30 FNU, bevorzugt kleiner als 20 FNU, und liegt besonders bevorzugt im Bereich zwischen 0,5 FNU und 2 FNU, gemessen durch Streulichtmessung mit Infrarotlicht nach den Vorschriften der Norm ISO 7027 bei einer Verdünnung des Solubilisats im Verhältnis 1:1000 in Wasser.

Je nach Anwendungsfall kann das Solubilisat gemäß der Erfindung bis zu 5 Gew.-% Wasser und/oder zwischen 12 Gew.-% und 20 Gew.-% Glycerin enthalten.

Es hat sich vorteilhafterweise herausgestellt, daß das erfindungsgemäße Solubilisat in einfacher Form in Kapseln zur oralen Einnahme zur Verfügung gestellt werden kann, da es die Kasein nicht angreift. Die Erfindug stellt damit auch eine Kapsel gefüllt mit dem Solubilisat zur Verfügung, wobei die Kapsel als Weichgelatinekapsel oder Hartgelatinekapsel oder als weiche, gelatinefreie Kapsel oder als harte, gelatinefreie Kapsel ausgebildet sein kann.

Eine weitere Darreichungsform ist ein Fluid enthaltend das erfindungsgemäße Solubilisat wobei das FLuid ein Lebensmittel, ein Getränke, ein kosmetikisches Produkt wie insbesondere eine Creme, Lotion oder Salbe oder ein pharmazeutisches Produkte sein kann. Insbesondere kann das Fluid eine wässrige Verdünnung des Solubilisats umfassen. Die Anwendbarkeit des erfindungsgemäßen Solubilisats in einem Fluid ist nicht an dessen Viskosität gebunden, ebenso kann das Solubilisat sowohl in hydrophile als auch in lipophile Medien eingearbeitet werden.

Im folgenden werden Ausführungsbeispiele für Solubilisate gemäß der Erfindung erläutert.

Die Partikelgrößenmessungen wurden mit dem ParticleMetrix NANOTRAC Rückstreu-Teilchenanalysator durchgeführt. Das Messprinzip beruht auf der dynamischen Lichtstreuung (DLS) in einer 180° Heterodyn-Rückstreuanordnung. Bei dieser Geometrie wird zum gestreuten Licht ein Teil des Laserstrahls dazu gemischt. Dies hat denselben positiven Effekt hinsichtlich des Signal/Rausch-Verhältnisses wie die Überlagerung aller Lichtwellenlängen in einem Fourier-Spektrometer. Die Farbe der Probe hat keinen Einfluss auf die Qualität der Messung. Die Messungen wurden in 1000facher wäßriger Verdünnung durchgeführt. Das Solubilisat wurde dazu unter Rühren in Wasser gelöst. Es ist vollständig klar in Wasser löslich. Diese Lösung ist stabil und transparent.

### Beispiel 1: Curcumin-Solubilisat mit Polysorbat 80

Zur Herstellung des Solubilisats werden allein 930 g Polysorbat 80 und 70 g 95%iges Curcumin-Pulver eingesetzt. In diesen 70 g sind 95 % Curcumin enthalten, also 66,5 g.

Als Polysorbat 80 sind handelsübliche Präparate wie beispielsweise TEGO SMO 20 V, InCoPa oder Crillet 4/Tween 80-LQ-(SG), Croda oder Lamesorb SMO 80, Cognis verwendbar. Als 95%iges Curcumin-Pulver können ebenfalls handelsübliche Präparate eingesetzt werden, beispielsweise Oleoresin Turmeric 95% (Curcumin powder), Jupiter Leys oder Curcumin BCM-95-SG, Eurochem oder Curcumin BCM-95-CG, Eurochem oder Curcuma Oleoresin 95%, Henry Lamotte.

Das Polysorbat 80 wird auf etwa 48 °C bis etwa 52 °C erwärmt. Unter Rühren wird das Curcuminpulver langsam zum Polysorbat 80 zugegeben. Während der Zugabe des Curcuminpulvers wird weiter auf etwa 87 °C bis etwa 91 °C erwärmt. Das entstehende Solubilisat wird auf unterhalb etwa 60 °C abgekühlt und ist dann bereit zur Abfüllung.

Das Solubilisat ist gelb-orange bis rötlich, sehr dunkel und farbintensiv sowie transparent.

Der Curcumin-Anteil kann auf Kosten des Polysorbat 80-Anteils auf bis zu etwa 10 Gew.-% gesteigert werden.

### Beispiel 2: Curcumin-Solubilisat mit Polysorbat 20

Zur Herstellung des Solubilisats werden allein 894 g Polysorbat 80 und 106 g 95%iges Curcumin-Pulver eingesetzt. In diesen 106 g sind 95 % Curcumin enthalten, also 100,7 g.

Als Polysorbat 20 sind handelsübliche Präparate wie beispielsweise TEGO SML 20 V, InCoPa oder Tween 20, Crillet 1-LQ-(SG), Croda oder Lamesorb SML 20, Cognis verwendbar. Als 95%iges Curcumin-Pulver können ebenfalls handelsübliche Präparate eingesetzt werden, beispielsweise Oleoresin Turmeric 95% (Curcumin powder), Jupiter Leys oder Curcumin BCM-95-SG, Eurochem oder Curcumin BCM-95-CG, Eurochem oder Curcuma Oleoresin 95%, Henry Lamotte.

Das Polysorbat 20 wird auf etwa 63 °C bis etwa 67 °C erwärmt. Unter Rühren wird das Curcuminpulver langsam zum Polysorbat 80 zugegeben. Während der Zugabe des Curcuminpulvers wird weiter auf etwa 83 °C bis etwa 87 °C erwärmt. Das entstehende Solubilisat wird langsam auf unterhalb etwa 45 °C abgekühlt und ist dann bereit zur Abfüllung.

Das Solubilisat ist gelb-orange bis rötlich, sehr dunkel und farbintensiv sowie transparent.

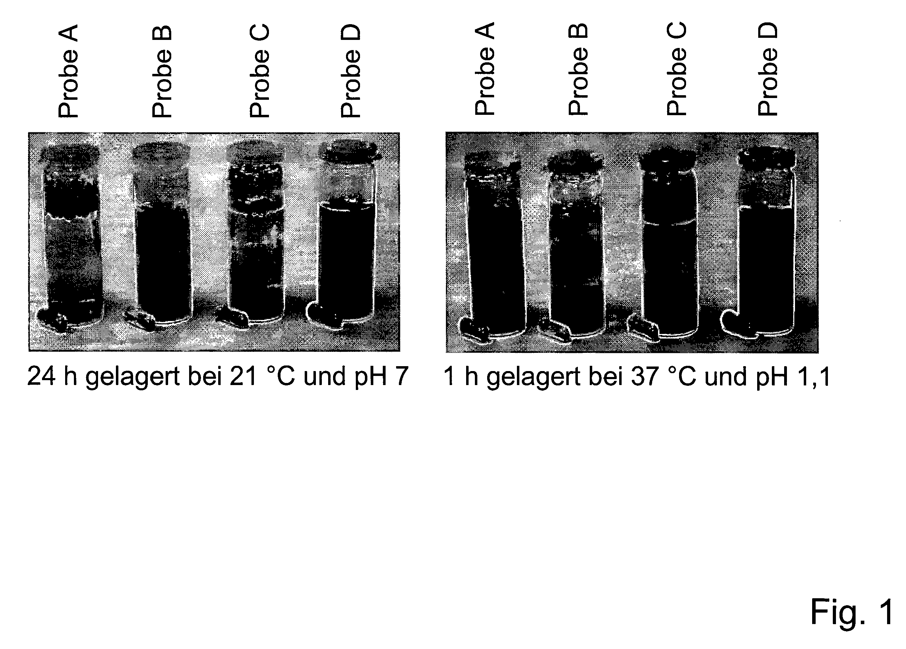

Der Curcumin-Anteil kann auf Kosten des Polysorbat 20-Anteils variiert werden.
In der beigefügten Figur 1 sind Fotografien von Proben verschiedener Curcumin-Formulierungen gezeigt. Auf der linken Seite der Abbildung sind die Proben 24 Stunden nach Zugabe zu Wasser bei einer Temperatur von 21°C unter neutralen Bedinungen (pH 7) gezeigt. Auf der rechten Seite der Abbildung sind die Proben unter physiologischen Bedingungen 1 Stunde nach Zugabe zu Wasser bei einer Temperatur von 37°C unter Bedingungen wie im Magen (pH 1,1) gezeigt. Es wurdejeweils soviel Wasser zu den Ausgangspräpraten zugegeben, daß die Curcuminkonzentration in der dargestellten Probe bei 11,4 g/L lag.

Im Einzelnen wurden folgende Formulierungen verglichen:

### Probe A

Curcumin-Extrakt, native Form, Pulver, 95 % Curcumin (BCM 95). 12 g des Pulvers wurden in 1 Liter Wasser eingebracht.

### Probe B

17,5 %iges Curcumin-Mikronisat, Pulver, Raps. 65,1 g des Pulvers wurden in 1 Liter Wasser eingebracht.

### Probe C

13%ige Curcumin-Formulierung, Pulver, Wacker Chemie. 87,7 g des Pulvers wurden in 1 Liter Wasser eingebracht.

### Probe D

Erfindungsgemäßes Curcuminsolubilisat nach Beispiel 1. 200 g des flüssigen Solubilisats wurden in 1 Liter Wasser eingebracht.

Während sowohl bei Raumtemperatur unter neutralen Bedingungen als auch bei 37°C und sauren Bedingungen die Proben A, B und C sowohl Sedimentbildung als auch Phasenseparation erkennen lassen, ist Probe D klar und homogen. Das erfindungsgemäße Solubilisat zeigt darüber hinaus im gesamten Temperaturbereich von -20°C bis 100°C keine Phasenseparation und keine Sedimentbildung. Darüber hinaus zeigt Probe D sowohl bei Raumtemperatur unter neutralen Bedingungen als auch bei 37°C und sauren Bedingungen eine intensivere Rotfärbung, wodurch die Probe als Schwarz-Weiß-Aufnahme deutlich dunkler erscheint als die Proben A, B und C, welche gelblich-orange gefärbt sind.

Es ist dem Fachmann ersichtlich, dass die Erfindung nicht auf die vorstehend beschriebenen Ausführungsbeispiele beschränkt ist, sondern vielmehr in vielfältiger Weise variiert werden kann. Insbesondere können die Merkmale der einzelnen Ausführungsbeispiele auch miteinander kombiniert oder gegeneinander ausgetauscht werden.

## Patentansprüche

1. Solubilisat bestehend aus
Curcumin mit einem Anteil von kleiner oder gleich 10 Gew.-%, bevorzugt kleiner oder gleich 7,5 Gew.-%, besonders bevorzugt 6 Gew.-% und
zumindest einem Emulgator mit einem HLB-Wert im Bereich zwischen 13 und 18, nämlich Polysorbat 80 oder Polysorbat 20 oder einer Mischung aus Polysorbat 20 und Polysorbat 80,
wobei der mittlere Durchmesser der mit Curcumin beladenen Micellen zwischen 5 nm und 40 nm, bevorzugt zwischen 6 nm und 20 nm, besonders bevorzugt zwischen 7 nm und 10 nm liegt.

2. Solubilisat nach Anspruch 1,
**dadurch gekennzeichnet, dass**
die Breite der Durchmesserverteilung der Micellen von etwa 4 nm bis etwa 30 nm reicht.

3. Solubilisat nach einem der vorangegangenen Ansprüche,
**dadurch gekennzeichnet, dass**
die Gesamt-Curcuminoid-Konzentration im humanen Blutplasma gemessen eine Stunde nach oraler Gabe von 500 mg Curcumin in Form des Solubilisats nach einem der vorangegangenen Ansprüche bei etwa 500 ng Curcuminoid pro mL Plasma ± 100 ng Curcuminoid pro mL Plasma liegt.

4. Solubilisat nach einem der vorangegangenen Ansprüche,
**dadurch gekennzeichnet, dass**
die Gesamt-Curcuminoid-Konzentration im humanen Blutplasma über einen Zeitraum von 24 Stunden gemessen als Fläche unter der Kurve der gesamten Curcumin-Konzentration im Blutplasma (area under the total curcumin plasma concentration-time curve AUC) im Bereich von etwa 9.500 bis etwa 10.000 nmol h /L liegt.

5. Solubilisat nach einem der vorangegangenen Ansprüche,
**dadurch gekennzeichnet, dass**
die Trübung des Solubilisats kleiner als 30 FNU, bevorzugt kleiner als 20 FNU, ist und besonders bevorzugt im Bereich zwischen 0,5 FNU und 2 FNU liegt, gemessen durch Streulichtmessung mit Infrarotlicht nach den Vorschriften der Norm ISO 7027 bei einer Verdünnung des Solubilisats im Verhältnis 1:1000 in Wasser.

6. Solubilisat nach einem der vorangegangenen Ansprüche,
**dadurch gekennzeichnet, dass**
die Trübung des Solubilisats nach 24 Stunden Lagerung bei 21°C und pH 7 kleiner als 30 FNU, bevorzugt kleiner als 20 FNU, ist und besonders bevorzugt im Bereich zwischen 0,5 FNU und 2 FNU liegt, gemessen durch Streulichtmessung mit Infrarotlicht nach den Vorschriften der Norm ISO 7027 bei einer Verdünnung des Solubilisats im Verhältnis 1:1000 in Wasser.

7. Solubilisat nach einem der vorangegangenen Ansprüche, **dadurch gekennzeichnet, dass**
die Trübung des Solubilisats nach 1 Stunde Lagerung bei 37°C und pH 1,1 kleiner als 30 FNU, bevorzugt kleiner als 20 FNU, ist und besonders bevorzugt im Bereich zwischen 0,5 FNU und 2 FNU liegt, gemessen durch Streulichtmessung mit Infrarotlicht nach den Vorschriften der Norm ISO 7027 bei einer Verdünnung des Solubilisats im Verhältnis 1:1000 in Wasser.

8. Solubilisat nach einem der vorangegangenen Ansprüche,
**dadurch gekennzeichnet, dass**
das Solubilisat zusätzlich bis zu 5 Gew.-% Wasser enthält.

9. Solubilisat nach einem der vorangegangenen Ansprüche,
**dadurch gekennzeichnet, dass**
das Solubilisat zusätzlich zwischen 12 Gew.-% und 20 Gew.-% Glycerin enthält.

10. Kapsel gefüllt mit einem Solubilisat nach einem der vorangegangenen Ansprüche,
**dadurch gekennzeichnet, dass**
die Kapsel als Weichgelatinekapsel oder Hartgelatinekapsel oder als weiche, gelatinefreie Kapsel oder als harte, gelatinefreie Kapsel ausgebildet ist.

11. Fluid enthaltend ein Solubilisat nach einem der Ansprüche 1 bis 10,
**dadurch gekennzeichnet, dass**
das Fluid aus der Gruppe ausgewählt ist, welche Lebensmittel, Getränke, Kosmetika und pharmazeutische Produkte umfaßt.

12. Fluid nach Anspruch 11,
**dadurch gekennzeichnet, dass**
das Fluid eine wässrige Verdünnung des Solubilisats umfaßt.

## Claims

1. Solubilisate composed of
curcumin in a quantity of less than or equal to 10% by weight, preferably less than or equal to 7.5% by weight, particularly preferably 6% by weight, and
at least one emulsifier having an HLB value in the range between 13 and 18, namely polysorbate 80 or polysorbate 20 or a mixture of polysorbate 20 and polysorbate 80,
wherein the average diameter of the micelles loaded with curcumin Is between 5 nm and 40 nm, preferably between 6 nm and 20 nm, particularly preferably between 7 nm and 10 nm.

2. Solubilisate according to Claim 1,
**characterized in that**
the with of the diameter distribution of the micelles ranges from approximately 4 nm to approximately 30 nm.

3. Solubilisate according to one of the preceding claims,
**characterized in that**
the total curcuminoid concentration in human blood plasma, measured one hour after oral administration of 500 mg curcumin in the form of the solubillsate according to one of the preceding claims, is approximately 500 ng curcuminoid per mL plasma ± 100 ng curcuminoid per mL plasma,

4. Solubilisate according to one of the preceding claims,
**characterized in that**
the total curcuminoid concentration in human blood plasma, measured over a period of 24 hours as the area under the curve of the total curcumin-concentration in blood plasma (area under the total curcumin plasma concentration-time curve AUC), is in the range of approximately 9,500 to approximately 10,000 nmol h/L.

5. Solubilisate according to one of the preceding claims,
**characterized In that**
the turbidity of the solubilisate is less than 30 FNU, preferably less than 20 FNU, and particularly preferably is in the range between 0.5 FNU and 2 FNU, determined by scattered light measurement with Infrared light according to the requirements of the ISO 7027 standard at a dilution of the solubilisate in water In a ratio of 1:1000.

6. Solubilisate according to one of the preceding claims,
**characterized In that**
the turbidity of the solubilisate after 24 hours storage at 21°C and pH 7 is less than 30 FNU, preferably less than 20 FNU, and particularly preferably is in the range between 0.5 FNU and 2 FNU, determined by scattered light measurement with infrared light according to the requirements of the ISO 7027 standard at a dilution of the solubilisate in water in a ratio of 1:1000.

7. Solubilisate according to one of the preceding claims,
**characterized in that**
the turbidity of the solubilisate after 1 hour storage at 37°C and pH 1.1 is less than 30 FNU, particularly preferably less than 20 FNU, and particularly preferably is In the range between 0.5 FNU and 2 FNU, determined by scattered light measurement with infrared light according to the requirements of the ISO 7027 standard at a dilution of the solubilisate in water In a ratio of 1:1000.

8. Solubilisate according to one of the preceding claims,
**characterized in that**
the solubilisate contains up to 5% by weight water.

9. Solubilisate according to one of the preceding claims,
**characterized in that**
the solubilisate contain between 12% by weight and 20% by weight glycerin.

10. Capsule filled with a solubilisate according to one of the preceding claims,
**characterized in that**
the capsule is formed as a soft gelatin capsule or hard gelatin capsule, or as a soft gelatin-free capsule or as a hard gelatin-free capsule.

11. Fluid containing a solubilisate according to one of Claims 1 to 10,
**characterized in that**
the fluid is selected from the group comprising foods, beverages, cosmetics, and pharmaceutical products.

12. Fluid according to Claim 11,
**characterized in that**
the fluid comprises an aqueous dilution of the solubilisate.

## Revendications

1. Solubilisat constitué de :
curcumine en une proportion inférieure ou égale à 10 % en poids, de préférence inférieure ou égale à 7,5 % en poids, particulièrement de préférence de 6 % en poids, et
au moins un émulsifiant ayant une valeur HLB dans la plage située entre 13 et 18, notamment le Polysorbat 80 ou le Polysorbat 20 ou un mélange de Polysorbat 20 et de Polysorbat 80,
dans lequel le diamètre moyen des micelles chargées de curcumine est situé entre 5 nm et 40 nm, de préférence entre 6 nm et 20 nm, particulièrement de préférence entre 7 nm et 10 nm.

2. Solubilisant selon la revendication 1, **caractérisé en ce que** la largeur de la distribution du diamètre des micelles va d'environ 4 nm à environ 30 nm,

3. Solubilisat selon l'une des revendications précédentes, **caractérisé en ce que** la concentration totale en curcuminoïde dans le plasma sanguin humain mesurée une heure après l'administration orale de 500 mg de curcumine sous la forme du solubilisat selon l'une des revendications précédentes se situe aux environs de 500 ng de curcuminoïde par ml de plasma ± 100 ng de curcuminolde par ml de plasma.

4. Solubilisat selon l'une des revendications précédentes, **caractérisé en ce que** la concentration totale en curcuminoïde dans le plasma sanguin humain pendant une durée de 24 heures, mesurée en tant qu'aire sous la courbe de la concentration totale en curcuminoïde dans le plasma sanguin (aire sous la courbe de temps - concentration totale plasmatique de curcumine AUC) se situe dans la plage d'environ 9 500 à environ 10 000 nmol h/l.

5. Solubilisat selon l'une des revendications précédentes, **caractérisé en ce que** la turbidité du solubilisat est inférieure à 30 FNU, de préférence inférieure à 20 FNU, et particulièrement de préférence dans la plage située entre 0,5 FNU et 2 FNU, mesurée par néphélométrie infrarouge d'après les prescriptions de la norme ISO 7027 à une dilution du solubilisat en un rapport de 1:1 000 dans de l'eau.

6. Solubilisat selon l'une des revendications précédentes, **caractérisé en ce que** la turbidité du solubilisat au bout de 24 heures de stockage à 21°C et à un pH de 7 est inférieure à 30 FNU, de préférence inférieure à 20 FNU, et particulièrement de préférence dans la plage située entre 0,5 FNU et 2 FNU, mesurée par néphélométrie infrarouge d'après les prescriptions de la norme ISO 7027 à une dilution du solubilisat en un rapport de 1:1 000 dans de l'eau.

7. Solubilisat selon l'une des revendications précédentes, **caractérisé en ce que** la turbidité du solubilisat au bout de 1 heure de stockage à 37°C et à un pH de 1,1 est inférieure à 30 FNU, de préférence inférieure à 20 FNU, et particulièrement de préférence dans la plage située entre 0,5 FNU et 2 FNU, mesurée par néphélométrie infrarouge d'après les prescriptions de la norme ISO 7027 à une dilution du solubilisat en un rapport de 1:1 000 dans de l'eau.

8. Solubilisat selon l'une des revendications précédentes, le solubilisat étant **caractérisé en ce qu'**il comprend en plus jusqu'à 5 % en poids d'eau.

9. Solubilisat selon l'une des revendications précédentes, le solubilisat étant **caractérisé en ce qu'**il contient en plus entre 12 % en poids et 20 % en poids de glycérine.

10. Gélule chargée d'un solubilisat selon l'une des revendications précédentes, la gélule étant **caractérisée en ce qu'**elle est conçue sous la forme d'une gélule de gélatine molle ou de gélatine dure ou sous la forme d'une gélule molle sans gélatine, ou d'une gélule dure sans gélatine.

11. Fluide contenant un solubilisat selon l'une des revendications 1 à 10, le fluide tant **caractérisé en ce qu'**il est choisi dans le groupe comprenant les produits alimentaires, les boissons, les cosmétiques, et les produits pharmaceutiques.

12. Fluide selon la revendication 11, le fluide étant **caractérisé en ce qu'**il comprend une dilution aqueuse du solubilisat.
